# EUROPEAN PATENT SPECIFICATION

(11) **EP 4 467 076 B1**
(45) Date of publication and mention of the grant of the patent: **22.04.2026**
(21) Application number: 22922121.3
(22) Date of filing: 12.12.2022
(51) Int. Cl.: G06T 7/00, A61B 6/50, A61B 6/00, A61B 6/02, A61B 6/46

(54) **IMAGE PROCESSING DEVICE, IMAGE PROCESSING METHOD, AND PROGRAM**
BILDVERARBEITUNGSVORRICHTUNG, BILDVERARBEITUNGSVERFAHREN UND PROGRAMM
DISPOSITIF DE TRAITEMENT D'IMAGE, PROCÉDÉ DE TRAITEMENT D'IMAGE ET PROGRAMME

(30) Priority: 19.01.2022 JP 2022006669
(43) Date of publication of application: 27.11.2024
(73) Proprietor: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2022/045733
(87) International publication number: WO 2023/139970

(56) References cited:
- JP-A- 2003 502 129
- JP-A- 2005 177 037
- JP-A- H07 299 053
- US-A- 5 491 627
- US-A1- 2006 147 101
- US-A1- 2009 080 752
- US-A1- 2011 103 673
- US-A1- 2011 103 673
- US-A1- 2012 294 502
- US-A1- 2012 294 502
- US-A1- 2017 200 267

## Description

### BACKGROUND OF THE INVENTION

### 1. Field of the Invention

The present disclosure relates to an image processing apparatus, an image processing method, and a program.

### 2. Description of the Related Art

A technique for recognizing a tissue of a breast in which calcification may occur by using a radiation image obtained by irradiating the breast with radiations is known. In addition, tomosynthesis imaging in which a series of a plurality of projection images is acquired by irradiating a breast with radiations having a plurality of angles is known. By reconfiguring the plurality of projection images obtained by tomosynthesis imaging, a plurality of tomographic images in which an overlap of mammary glands is reduced are obtained. Further, a technique of generating one synthesized two-dimensional image in which an overlap of mammary glands is reduced by synthesizing a plurality of tomographic images is known.

JP2020-096752A discloses a technique of detecting a position of a lesion, such as calcification of a breast, in a plurality of tomographic images generated by reconstructing a plurality of projection images obtained by tomosynthesis imaging, increasing a weight such that the detected lesion is emphasized, and generating a synthesized two-dimensional image.

Documents US2011/103673A1, US2017/200267A1 and US5491627A also disclose similar automated calcification detection schemes.

### SUMMARY OF THE DISCLOSURE

In the tomographic image, noise that is indistinguishable from a small calcification image is present. It is difficult for a person to visually distinguish a small calcification image and noise on a tomographic image, and it is also difficult to mechanically separate a small calcification image and noise on a tomographic image. In addition, in a case where a synthesized two-dimensional image is generated based on the plurality of tomographic images, noise is drawn on the synthesized two-dimensional image as a pseudo calcification image. As a result, this may lead to misdiagnosis.

JP2020-096752A discloses a method of increasing a weight such that a lesion such as calcification is emphasized in a plurality of tomographic images and generating a synthesized two-dimensional image. However, JP2020-096752A does not disclose a method of distinguishing a calcification image and noise.

An object of the technology of the present disclosure is to provide an image processing apparatus, an image processing method, and a program that can accurately distinguish between a calcification image and noise.

In order to achieve the above object, according to the present disclosure, there is provided an image processing apparatus including: at least one processor, in which the processor is configured to execute: calcification candidate image detection processing of detecting a calcification candidate image estimated to be a calcification image from a series of a plurality of projection images obtained by tomosynthesis imaging of a breast or a plurality of tomographic images obtained from the plurality of projection images; calcification candidate image group generation processing of generating a calcification candidate image group by cutting out a region including the calcification candidate image detected by the calcification candidate image detection processing, from each of the plurality of projection images; and calcification determination processing of determining whether or not the calcification candidate image is a calcification image based on the calcification candidate image group generated by the calcification candidate image group generation processing.

Preferably, the processor is configured to: individually generate the calcification candidate image group for each of a plurality of the calcification candidate images in the calcification candidate image group generation processing, in a case where a plurality of the calcification candidate images are detected in the calcification candidate image detection processing.

Preferably, the processor is configured to: detect only the calcification candidate image of which a signal value is equal to or smaller than a certain value in the calcification candidate image detection processing.

Preferably, the processor is configured to: detect the calcification candidate image estimated to be a calcification image from the plurality of projection images in the calcification candidate image group generation processing; and generate the calcification candidate image group in the calcification candidate image group generation processing, in a case where a corresponding calcification candidate image is detected from a certain number or more of the projection images among the series of the plurality of projection images in the calcification candidate image detection processing.

Preferably, the processor is configured to: detect the calcification candidate image estimated to be a calcification image from the plurality of projection images in the calcification candidate image group generation processing; and perform weighting on the calcification candidate image based on a determination result of the calcification determination processing, and generate the plurality of tomographic images by a back projection method.

Preferably, the processor is configured to: detect the calcification candidate image estimated to be a calcification image from the plurality of tomographic images in the calcification candidate image group generation processing; and perform weighting on the calcification candidate image based on a determination result of the calcification determination processing, and generate a synthesized two-dimensional image by synthesizing the plurality of tomographic images.

Preferably, the processor is configured to: execute the calcification determination processing by inputting the calcification candidate image group into a machine-learned model obtained by performing machine learning of a relationship between the calcification candidate image group and whether or not the calcification candidate image is a calcification image.

Preferably, the processor is configured to: determine whether or not the calcification candidate image is a calcification image based on a pattern of signals included in each of the calcification candidate images included in the calcification candidate image group in the calcification determination processing.

According to the present disclosure, there is provided an image processing method including: a calcification candidate image detection step of detecting a calcification candidate image estimated to be a calcification image from a series of a plurality of projection images obtained by tomosynthesis imaging of a breast or a plurality of tomographic images obtained from the plurality of projection images; a calcification candidate image group generation step of generating a calcification candidate image group by cutting out a region including the calcification candidate image detected by the calcification candidate image detection step, from each of the plurality of projection images; and a calcification determination step of determining whether or not the calcification candidate image is a calcification image based on the calcification candidate image group generated by the calcification candidate image group generation step.

According to the present disclosure, there is provided a program causing a computer to execute: calcification candidate image detection processing of detecting a calcification candidate image estimated to be a calcification image from a series of a plurality of projection images obtained by tomosynthesis imaging of a breast or a plurality of tomographic images obtained from the plurality of projection images; calcification candidate image group generation processing of generating a calcification candidate image group by cutting out a region including the calcification candidate image detected by the calcification candidate image detection processing, from each of the plurality of projection images; and calcification determination processing of determining whether or not the calcification candidate image is a calcification image based on the calcification candidate image group generated by the calcification candidate image group generation processing.

According to the technology of the present disclosure, it is possible to provide an image processing apparatus, an image processing method, and a program that can accurately distinguish between a calcification image and noise.

### SUMMARY OF THE INVENTION

The invention is defined by the appended independent claims 1 (image processing apparatus), 8 (image processing method), and 9 (program).

### BRIEF DESCRIPTION OF THE DRAWINGS

Fig. 1 is a diagram illustrating an example of an entire configuration of a radiography system.
Fig. 2 is a diagram illustrating an example of tomosynthesis imaging.
Fig. 3 is a block diagram illustrating an example of a configuration of an image processing apparatus.
Fig. 4 is a block diagram illustrating an example of a function realized by a control unit of the image processing apparatus.
Fig. 5 is a diagram schematically illustrating a flow of processing by the image processing apparatus.
Fig. 6 is a diagram conceptually illustrating an example of processing of generating one calcification candidate image group.
Fig. 7 is a diagram conceptually illustrating an example of tomographic image generating processing.
Fig. 8 is a flowchart illustrating a flow of a series of processing by the image processing apparatus.
Fig. 9 is a diagram conceptually illustrating intensities of signals included in a tomographic image.
Fig. 10 is a diagram conceptually illustrating intensities of signals included in each of calcification candidate images included in a calcification candidate image group.
Fig. 11 is a diagram conceptually illustrating an example of learning processing of a machine learning model.
Fig. 12 is a diagram schematically illustrating a flow of processing by the image processing apparatus according to a first modification example.
Fig. 13 is a flowchart illustrating a flow of calcification candidate image group generation processing according to the first modification example.
Fig. 14 is a block diagram illustrating a function realized by a control unit of the image processing apparatus according to a second modification example.
Fig. 15 is a diagram schematically illustrating a flow of processing by the image processing apparatus according to the second modification example.
Fig. 16 is a diagram conceptually illustrating an example of synthesized two-dimensional image generation processing.
Fig. 17 is a flowchart illustrating a flow of a series of processing by the image processing apparatus according to the second modification example.

### DESCRIPTION OF THE PREFERRED EMBODIMENTS

Hereinafter, an embodiment of the present disclosure will be described in detail with reference to the drawings.

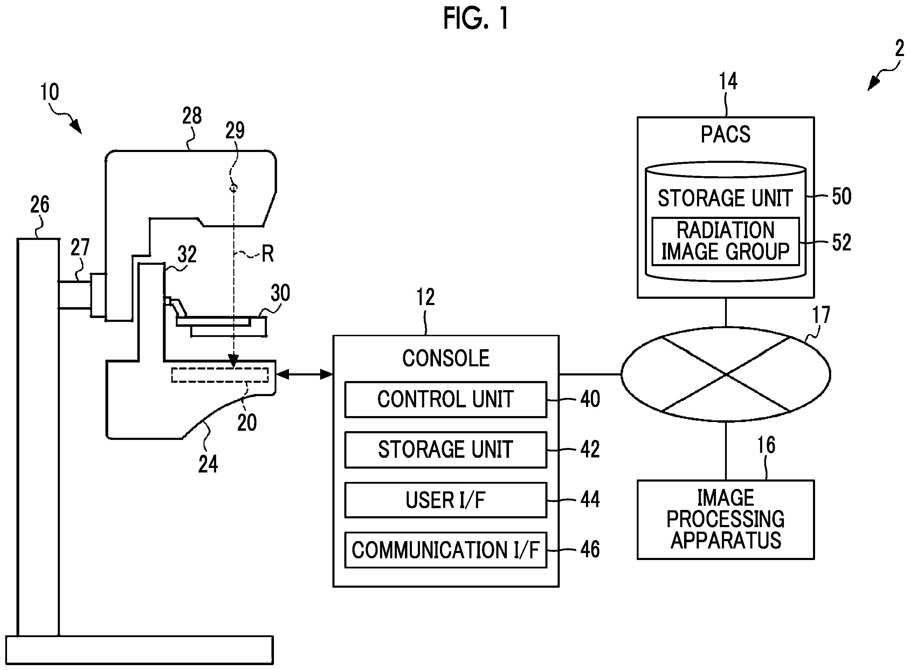

Fig. 1 illustrates an example of an entire configuration of a radiography system 2 according to the present embodiment. The radiography system 2 includes a mammography apparatus 10, a console 12, a picture archiving and communication systems (PACS) 14, and an image processing apparatus 16. The console 12, the PACS 14, and the image processing apparatus 16 are connected to each other via a network 17 by wired communication or wireless communication.

Fig. 1 illustrates an example of an appearance of the mammography apparatus 10. Fig. 1 illustrates an example of an appearance in a case where the mammography apparatus 10 is viewed from a left side of a subject.

The mammography apparatus 10 operates according to a control of the console 12, and is a radiography apparatus that acquires a radiation image of a breast M by irradiating the breast M of the subject as a target with radiations R (for example, X rays) from a radiation source 29.

The mammography apparatus 10 has a function of performing normal imaging in which imaging is performed in a state where the radiation source 29 is positioned at an irradiation position along a normal direction of a detection surface 20A of a radiation detector 20 and a function of performing tomosynthesis imaging in which imaging is performed in a state where the radiation source 29 is moved to each of a plurality of irradiation positions.

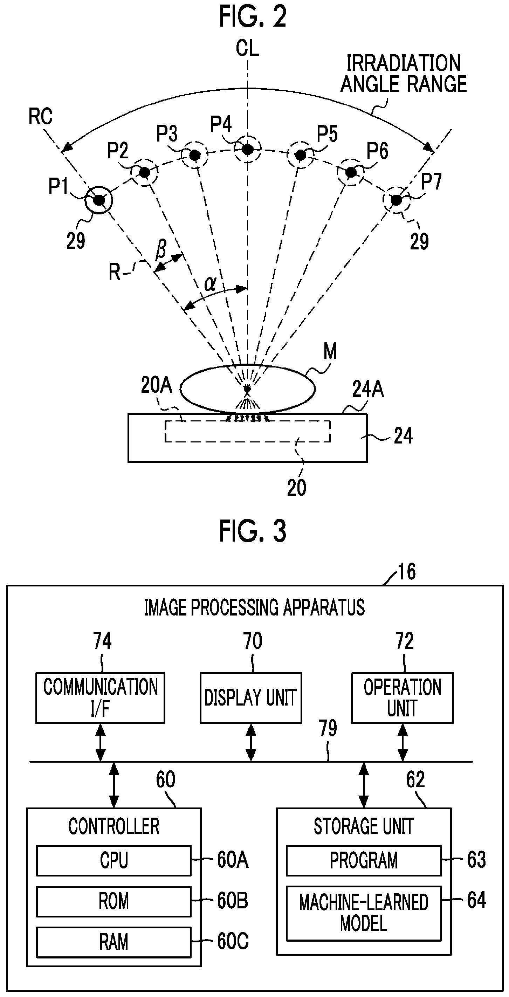

As illustrated in Fig. 1, the mammography apparatus 10 includes an imaging table 24, a base 26, an arm portion 28, and a compression unit 32. A radiation detector 20 is disposed inside the imaging table 24. As illustrated in Fig. 2, in the mammography apparatus 10, in a case of performing imaging, the breast M of the subject is positioned on an imaging surface 24A of the imaging table 24 by a user.

The radiation detector 20 detects radiations R passing through the breast M as a target. Specifically, the radiation detector 20 detects the radiations R that pass through the breast M of the subject, enter into the imaging table 24, and reach a detection surface 20A of the radiation detector 20, and generates a radiation image based on the detected radiations R. The radiation detector 20 outputs image data representing the generated radiation image. In the following, a series of operations of irradiating the breast with radiations R from the radiation source 29 and generating a radiation image by the radiation detector 20 may be referred to as "imaging". The radiation detector 20 may be an indirect-conversion-type radiation detector that converts the radiations R into light beams and converts the converted light beams into charges, or may be a direct-conversion-type radiation detector that directly converts the radiations R into charges.

A compression plate 30 that is used for compressing the breast M when performing imaging is attached to the compression unit 32. The compression plate 30 is moved in a direction toward or away from the imaging table 24 (hereinafter, referred to as a "vertical direction") by a compression plate driving unit (not illustrated) provided in the compression unit 32. The compression plate 30 compresses the breast M between the compression plate 30 and the imaging table 24 by moving in the vertical direction.

The arm portion 28 can be rotated with respect to the base 26 by a shaft portion 27. The shaft portion 27 is fixed to the base 26, and the shaft portion 27 and the arm portion 28 are rotated as one body. Gears are provided in each of the shaft portion 27 and the compression unit 32 of the imaging table 24. By switching the gears between an engaged state and a non-engaged state, the compression unit 32 of the imaging table 24 and the shaft portion 27 can be switched between a state where the compression unit 32 and the shaft portion 27 are connected to each other and are rotated as one body and a state where the shaft portion 27 is separated from the imaging table 24 and idles. Elements for switching between transmission and non-transmission of power of the shaft portion 27 are not limited to the gears, and various mechanical elements can be used. The arm portion 28 and the imaging table 24 can be separately rotated with respect to the base 26 with the shaft portion 27 as a rotation axis.

In a case of performing tomosynthesis imaging in the mammography apparatus 10, the radiation source 29 is sequentially moved to each of a plurality of irradiation positions having different irradiation angles by rotation of the arm portion 28. The radiation source 29 includes a radiation tube (not illustrated) that generates the radiations R, and the radiation tube is moved to each of the plurality of irradiation positions in accordance with the movement of the radiation source 29.

Fig. 2 illustrates an example of tomosynthesis imaging. In Fig. 2, the compression plate 30 is not illustrated. In the present embodiment, the radiation source 29 is moved to irradiation positions Pk (k = 1, 2, ..., 7) at which irradiation angles are different by a certain angle β. That is, the radiation source 29 is sequentially moved to a plurality of positions at which the irradiation angles of the radiations R with respect to the detection surface 20A of the radiation detector 20 are different. In Fig. 2, the number of the irradiation positions Pk is set to 7. On the other hand, the number of the irradiation positions Pk is not limited and can be changed as appropriate.

At each irradiation position Pk, the radiation R is emitted from the radiation source 29 toward the breast M, and the radiation detector 20 generates a radiation image by detecting the radiation R passing through the breast M. In the radiography system 2, in a case where the radiation source 29 is moved to each of the irradiation positions Pk and tomosynthesis imaging for generating a radiation image at each irradiation position Pk is performed, in the example of Fig. 2, seven radiation images are obtained.

In the following, in the tomosynthesis imaging, the radiation image obtained by performing imaging at each irradiation position Pk is referred to as a "projection image" in a case of distinguishing and describing the radiation image from a tomographic image, and a plurality of projection images obtained by performing tomosynthesis imaging once are referred to as a "series of the plurality of projection images". Further, in a case where the projection image is referred to without distinguishing the projection image from the tomographic image, the projection image is simply referred to as a "radiation image".

In addition, as illustrated in Fig. 2, the irradiation angle of the radiation R means an angle α formed by a normal line CL of the detection surface 20A of the radiation detector 20 and a radiation axis RC. The radiation axis RC means an axis connecting a focus of the radiation source 29 at each irradiation position Pk and a preset position. Further, the detection surface 20A of the radiation detector 20 is a surface substantially parallel to the imaging surface 24A. The radiation R emitted from the radiation source 29 is a cone beam having a focus as the apex and the radiation axis RC as a central axis.

On the other hand, in a case of performing normal imaging in the mammography apparatus 10, the position of the radiation source 29 is fixed to the irradiation position P4 at which the irradiation angle α is 0 degree. The radiation R is emitted from the radiation source 29 according to an instruction of the console 12, and the radiation detector 20 generates a radiation image by detecting the radiation R passing through the breast M.

The mammography apparatus 10 and the console 12 are connected to each other by wired communication or wireless communication. The radiation image generated by the radiation detector 20 in the mammography apparatus 10 is output to the console 12 by wired communication or wireless communication via a communication interface (I/F) (not illustrated).

The console 12 includes a control unit 40, a storage unit 42, a user I/F 44, and a communication I/F 46. As described above, the control unit 40 has a function of performing control related to radiography by the mammography apparatus 10. The control unit 40 is configured with, for example, a computer system including a central processing unit (CPU), a read only memory (ROM), and a random access memory (RAM).

The storage unit 42 stores information related to radiography, the radiation image acquired from the mammography apparatus 10, and the like. The storage unit 42 is a non-volatile storage such as a hard disk drive (HDD) or a solid state drive (SSD).

The user I/F 44 includes an input device including various buttons and switches, which are related to imaging of the radiation image and are operated by a user such as a technician, and a display device such as a lamp or a display that displays information related to imaging, the radiation image obtained by imaging, and the like.

The communication I/F 46 performs communication of various types of data such as the information related to radiography, the radiation image, and the like between the console 12 and the mammography apparatus 10 by wired communication or wireless communication. Further, the communication I/F 46 performs communication of various types of data such as the radiation image between the PACS 14 and the image processing apparatus 16 via the network 17 by wired communication or wireless communication.

In addition, the PACS 14 includes a storage unit 50 (refer to Fig. 1) that stores a radiation image group 52. The radiation image group 52 includes a projection image acquired from the console 12 via the network 17.

The image processing apparatus 16 has a function of supporting diagnosis by a doctor by performing determination related to diagnosis of a lesion in a case where a doctor or the like (hereinafter, simply referred to as a "doctor") performs diagnosis related to a lesion of the breast M using the radiation image.

Fig. 3 illustrates an example of a configuration of the image processing apparatus 16. The image processing apparatus 16 includes a control unit 60, a storage unit 62, a display unit 70, an operation unit 72, and a communication I/F 74. The control unit 60, the storage unit 62, the display unit 70, the operation unit 72, and the communication I/F 74 are connected to each other via a bus 79 such as a system bus or a control bus such that various types of information can be exchanged.

The control unit 60 controls overall operations of the image processing apparatus 16. The control unit 60 is configured with a computer system including a CPU 60A, a ROM 60B, and a RAM 60C. Various programs, data, and the like for performing control by the CPU 60A are stored in advance in the ROM 60B. The RAM 60C temporarily stores various types of data.

The storage unit 62 is a non-volatile storage such as an HDD or an SSD. The storage unit 62 stores a program 63 for causing the control unit 60 to execute various types of processing, a machine-learned model 64 for performing calcification determination processing to be described later, and the like.

The display unit 70 is a display that displays a radiation image, various types of information, and the like. The operation unit 72 is used to allow a doctor to input an instruction for diagnosing a lesion of a breast using a radiation image, various types of information, and the like. The operation unit 72 includes, for example, various switches, a touch panel, a touch pen, a mouse, and the like.

The communication I/F 74 performs communication of various types of information between the console 12 and the PACS 14 via the network 17 by wireless communication or wired communication.

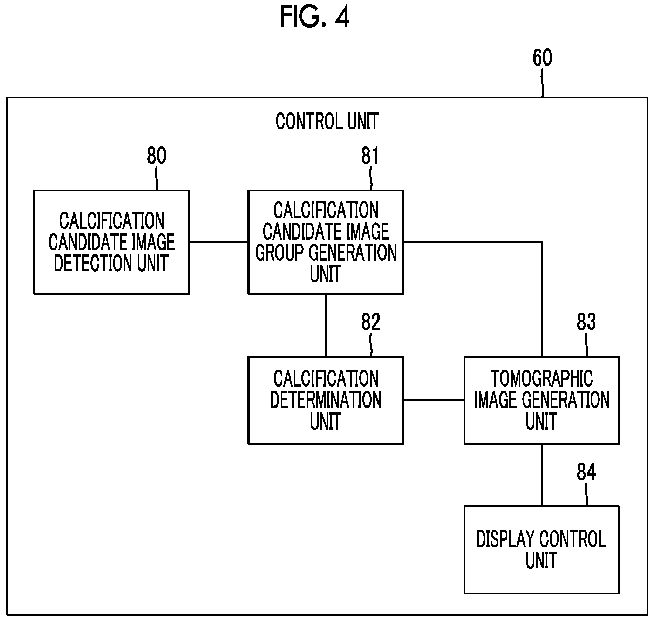

Fig. 4 illustrates an example of a function realized by the control unit 60 of the image processing apparatus 16. The CPU 60A of the control unit 60 realizes various functions by executing processing based on the program 63 stored in the storage unit 62. The control unit 60 functions as a calcification candidate image detection unit 80, a calcification candidate image group generation unit 81, a calcification determination unit 82, a tomographic image generation unit 83, and a display control unit 84.

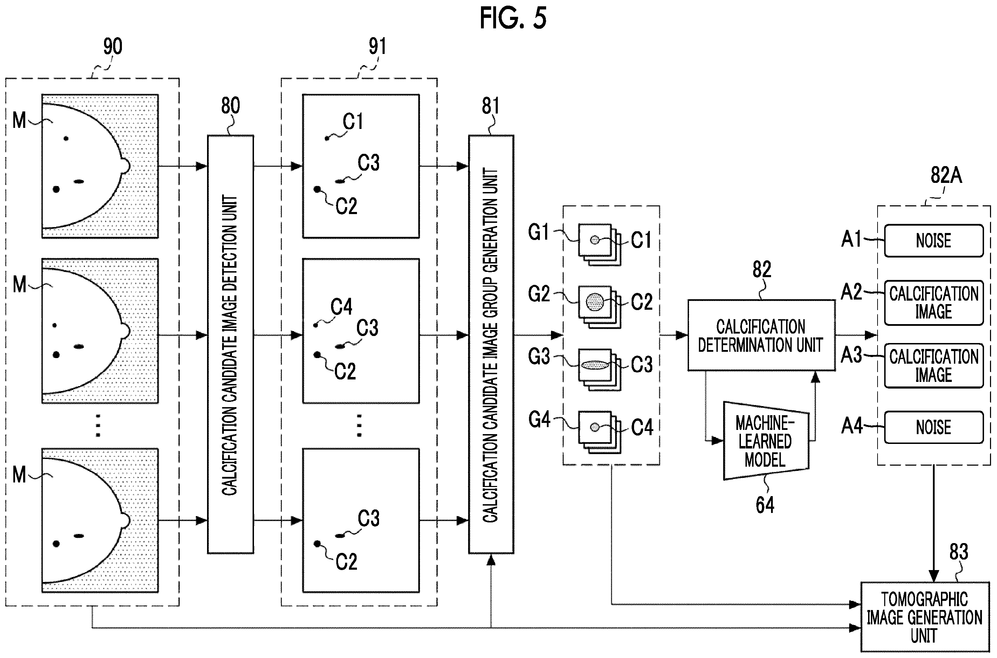

Fig. 5 schematically illustrates a flow of processing by the image processing apparatus 16. Processing by the calcification candidate image detection unit 80, the calcification candidate image group generation unit 81, the calcification determination unit 82, and the tomographic image generation unit 83 will be described with reference to Fig. 5.

The calcification candidate image detection unit 80 acquires a series of a plurality of projection images 90 from the console 12 of the mammography apparatus 10 or the PACS 14. The calcification candidate image detection unit 80 performs calcification candidate image detection processing of detecting, as a calcification candidate image, a region in the breast M that is estimated as an image of a tissue in which an occurrence of calcification is expected (hereinafter, referred to as a calcification image), from the series of the plurality of acquired projection images 90. Specifically, the calcification candidate image detection unit 80 detects a calcification candidate image from each of the series of the plurality of projection images 90.

As the calcification candidate image detection unit 80, a detector using a known computer-aided diagnosis (CAD) algorithm can be used. In the CAD algorithm, a probability (likelihood) indicating that a pixel in the projection image 90 is a calcification image is derived, and a pixel of which the probability is equal to or higher than a predetermined threshold value is detected as the calcification candidate image.

The calcification candidate image detection unit 80 is not limited to the detector using the CAD algorithm, and may be configured by a machine-learned model obtained by performing machine learning.

The detection result of the calcification candidate image by the calcification candidate image detection unit 80 is output as, for example, a mask image 91 representing a position of the calcification image. The mask image 91 is a binary image in which a pixel included in the calcification image is represented by "1" and the other pixels are represented by "0". The calcification candidate image detection unit 80 outputs one mask image 91 for each of the plurality of projection images 90. In the example illustrated in Fig. 5, four calcification candidate images C1 to C4 are detected by the calcification candidate image detection unit 80. Here, the calcification candidate images C1 and C4 are detected from only one projection image 90 among the plurality of projection images 90. The calcification candidate images C2 and C3 are detected from all of the projection images 90.

The calcification candidate image group generation unit 81 performs calcification candidate image group generation processing of generating a calcification candidate image group by cutting out regions respectively corresponding to the region including the calcification candidate image detected by the calcification candidate image detection processing, from each of the plurality of projection images 90.

The calcification candidate image group generation unit 81 generates a calcification candidate image group including a plurality of calcification candidate images by cutting out a region corresponding to the same calcification candidate image, from each of the series of the plurality of projection images 90, based on the mask image 91. In addition, in a case where a plurality of calcification candidate images are detected in the calcification candidate image detection processing, the calcification candidate image group generation unit 81 individually generates a calcification candidate image group for each of the calcification candidate images.

The calcification candidate image group generation unit 81 individually generates a calcification candidate image group for each of the four calcification candidate images C1 to C4. Thereby, a calcification candidate image group G1 including the calcification candidate image C1, a calcification candidate image group G2 including the calcification candidate image C2, a calcification candidate image group G3 including the calcification candidate image C3, and a calcification candidate image group G4 including the calcification candidate image C4 are generated.

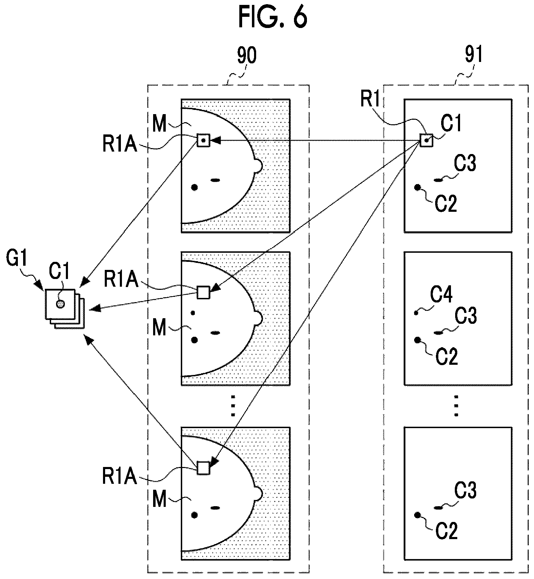

Fig. 6 conceptually illustrates an example of processing of generating the calcification candidate image group G1. The calcification candidate image group generation unit 81 specifies a region R1 in the mask image 91 that includes the calcification candidate image C1. In addition, the calcification candidate image group generation unit 81 specifies regions R1A respectively corresponding to the region R1 including the calcification candidate image C1 based on positional information of a radiation tube when each of the series of the plurality of projection images 90 is captured, and generates a calcification candidate image group G1 by cutting out the regions R1A from each of the plurality of projection images 90.

The calcification determination unit 82 performs calcification determination processing of determining whether or not the calcification candidate image is a calcification image based on the calcification candidate image group generated by the calcification candidate image group generation processing. In the present embodiment, the calcification determination unit 82 inputs the calcification candidate image group to the machine-learned model 64 obtained by performing machine learning on a relationship between the calcification candidate image and whether or not the calcification candidate image is a calcification image, and acquires a determination result (hereinafter, referred to as a calcification determination result) 82A output from the machine-learned model 64.

The machine-learned model 64 is, for example, a convolutional neural network (CNN) obtained by performing machine learning by deep learning. The machine-learned model 64 may perform two-dimensional convolution processing, pooling processing, and the like by treating the plurality of calcification candidate images included in the calcification candidate image group, as channels. In addition, the machine-learned model 64 may perform three-dimensional convolution processing, pooling processing, and the like by using the calcification candidate image group as voxel data.

In the example illustrated in Fig. 5, the calcification determination unit 82 individually inputs each of the four calcification candidate image groups G1 to G4 to the machine-learned model 64. From the machine-learned model 64, the calcification determination result 82A representing whether or not the calcification candidate image is a calcification image is output for each of the four calcification candidate image groups G1 to G4. The calcification determination result 82A includes determination results A1 to A4. The determination result A1 represents that the calcification candidate image C1 included in the calcification candidate image group G1 is not a calcification image but noise. The determination result A2 represents that the calcification candidate image C2 included in the calcification candidate image group G2 is a calcification image. The determination result A3 represents that the calcification candidate image C3 included in the calcification candidate image group G3 is a calcification image. The determination result A4 represents that the calcification candidate image C4 included in the calcification candidate image group G4 is not a calcification image but noise.

The tomographic image generation unit 83 performs tomographic image generation processing of performing weighting on the calcification candidate image based on the calcification determination result 82A by the calcification determination processing and then generating a plurality of tomographic images by a back projection method.

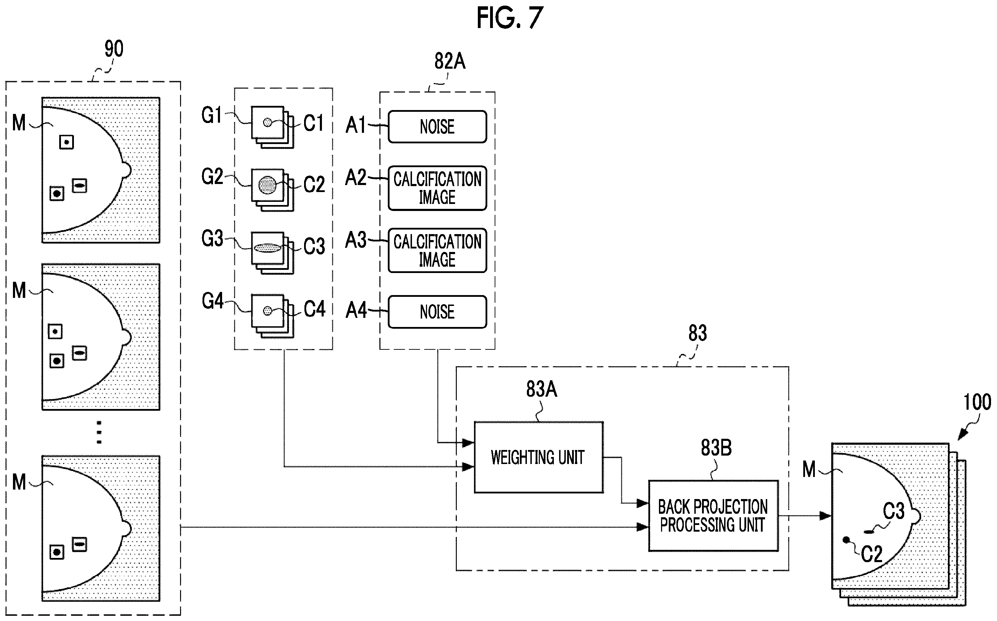

Fig. 7 conceptually illustrates an example of tomographic image generation processing by the tomographic image generation unit 83. As illustrated in Fig. 7, the tomographic image generation unit 83 includes a weighting unit 83A and a back projection processing unit 83B. The weighting unit 83A performs weighting on the calcification candidate image based on the calcification determination result 82A. Specifically, the weighting unit 83A increases a weight of a pixel included in the calcification candidate image determined to be a calcification image, and decreases a weight of a pixel included in the calcification candidate image determined to be noise.

In a state where the weighting unit 83A performs weighting on pixels of the series of the plurality of projection images 90 acquired by the calcification candidate image detection unit 80, the back projection processing unit 83B generates a plurality of tomographic images 100 having different heights from the imaging surface 24A by back projection. As the back projection method, a filter back projection (FBP) method, a successive approximation reconfiguration method, or the like can be used. The back projection processing unit 83B outputs the plurality of generated tomographic images 100 to the display control unit 84.

The display control unit 84 performs display processing of displaying the plurality of tomographic images 100 generated by the tomographic image generation processing on the display unit 70. The display control unit 84 may perform highlight display by coloring or the like the calcification candidate image determined to be a calcification image by the calcification determination processing.

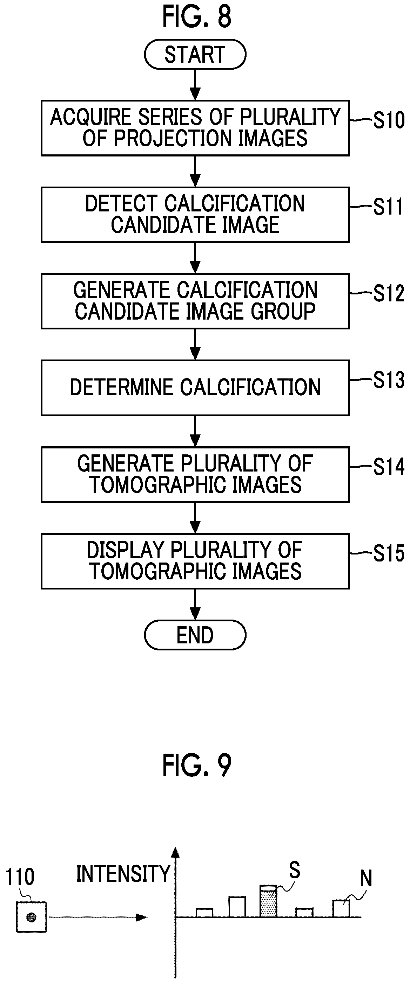

Next, a series of processing by the image processing apparatus 16 will be described with reference to Fig. 8. First, in step S10, the calcification candidate image detection unit 80 acquires a series of a plurality of projection images 90 from the console 12 of the mammography apparatus 10 or the PACS 14.

In step S11, the calcification candidate image detection unit 80 detects, as a calcification candidate image, a region estimated to be a calcification image from the series of the plurality of projection images 90 acquired in step S10.

In step S12, the calcification candidate image group generation unit 81 generates a calcification candidate image group by cutting out regions respectively corresponding to the region including the calcification candidate image detected in step S11, from each of the plurality of projection images 90.

In step S13, the calcification determination unit 82 determines whether or not the calcification candidate image is a calcification image based on the calcification candidate image group generated in step S12. Specifically, the calcification determination unit 82 inputs the calcification candidate image group to the machine-learned model 64, and acquires a calcification determination result 82A from the machine-learned model 64.

In step S14, the tomographic image generation unit 83 performs weighting on the calcification candidate image based on the calcification determination result 82A acquired in step S13, and then generates a plurality of tomographic images 100 by a back projection method.

In step S15, the display control unit 84 displays the plurality of tomographic images 100 generated in step S14 on the display unit 70.

As described above, according to the technology of the present disclosure, based on the calcification candidate image group generated by cutting out the regions respectively corresponding to the region including the calcification candidate image, from each of the plurality of projection images 90, it is determined whether or not the calcification candidate image is a calcification image, and thus, it is possible to accurately distinguish a calcification image and noise.

Fig. 9 conceptually illustrates intensities of signals included in one tomographic image 110 generated from the series of the plurality of projection images 90. As illustrated in Fig. 9, in the tomographic image 110, a calcification image signal S representing a calcification image is buried in a noise signal N. For this reason, in a case where the tomographic image 110 is used, it is difficult to accurately distinguish the calcification image and noise.

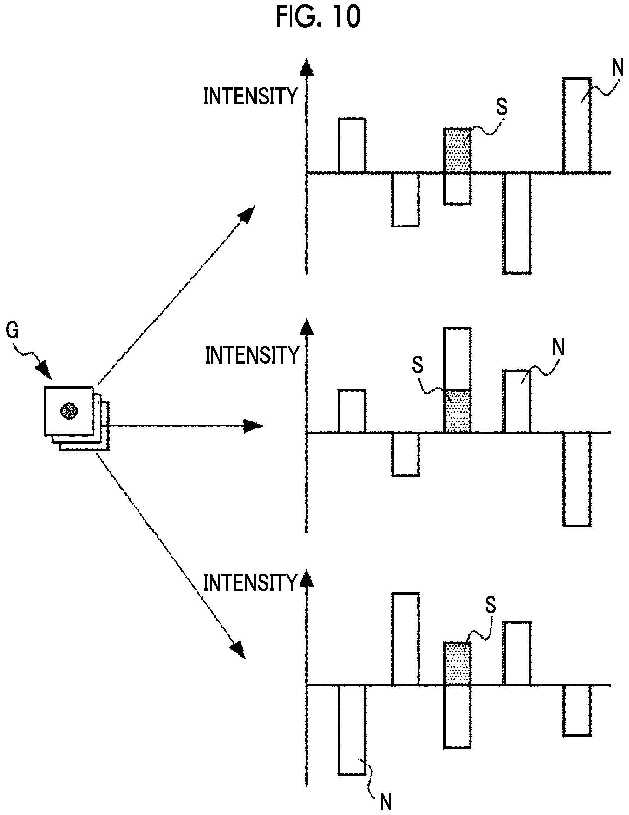

Fig. 10 conceptually illustrates intensities of signals included in each of the calcification candidate images included in the calcification candidate image group G As illustrated in Fig. 10, the noise signal N is randomly generated in each of the calcification candidate images, whereas the calcification image signal S is generated with a common intensity in each of the calcification candidate images. As described above, according to the technology of the present disclosure, the calcification candidate image group is used, and thus, the calcification image and the noise can be accurately distinguished based on a pattern of the signals between the calcification candidate images.

In the embodiment, the calcification candidate image detection unit 80 detects the calcification candidate image from the plurality of projection images 90. The calcification candidate image detection unit 80 may detect only a calcification candidate image (so-called pale calcification candidate image) of which a signal value is equal to or smaller than a certain value. This is because a shape of the pale calcification candidate image is not accurately represented and it is difficult to determine whether or not the tomographic image 100 as a clinical image that is displayed on the display unit 70 is a calcification image.

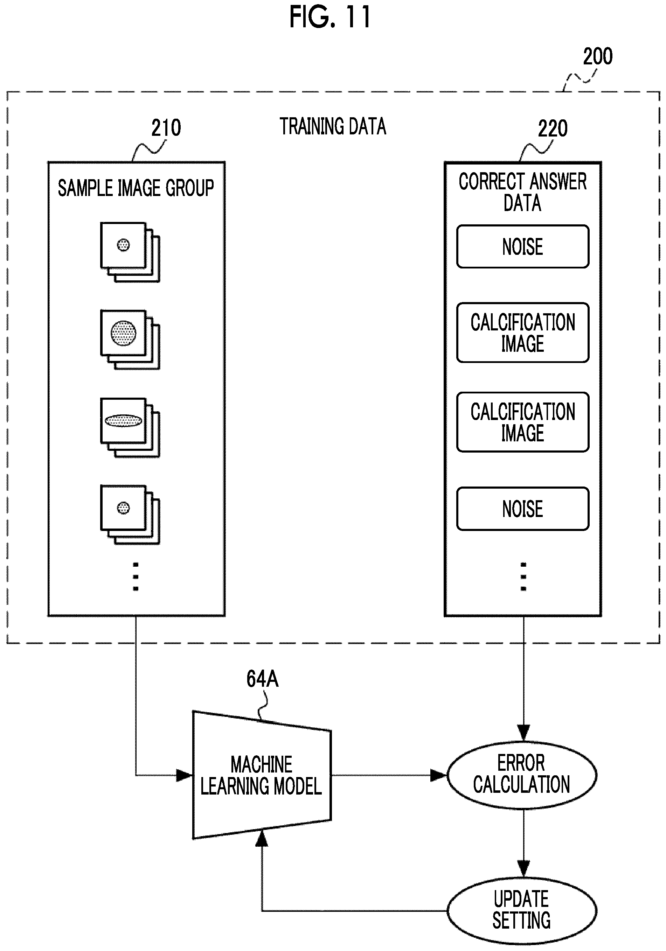

Fig. 11 conceptually illustrates an example of learning processing of a machine learning model 64A. As illustrated in Fig. 11, the machine-learned model 64 is generated by performing machine learning on the machine learning model 64A using training data 200 in a learning phase. The training data 200 includes a set of a plurality of sample image groups 210 and a plurality of pieces of correct answer data 220. The sample image group 210 is images corresponding to the calcification candidate image group. The correct answer data 220 is information representing a correct answer as to whether or not the calcification candidate image included in the sample image group 210 is a calcification image.

The machine learning is performed on the machine learning model 64A using, for example, an error back propagation method. In the learning phase, error calculation between the determination result obtained by inputting the sample image group 210 to the machine learning model 64A and the correct answer data 220 and update setting of weights and biases are repeatedly performed. The machine learning model 64A on which machine learning is performed in the learning phase is stored in the storage unit 62, as a machine-learned model 64. The machine learning of the machine learning model 64A may be performed in the image processing apparatus 16 or in an external apparatus.

Hereinafter, various modification examples of the embodiment will be described.

### [First Modification Example]

The first modification example is different from the embodiment only in calcification candidate image group generation processing by the calcification candidate image group generation unit 81. In the present modification example, the calcification candidate image group generation unit 81 generates a calcification candidate image group in a case where a corresponding calcification candidate image is detected from two or more projection images 90 among the series of the plurality of projection images 90 in the calcification candidate image detection processing.

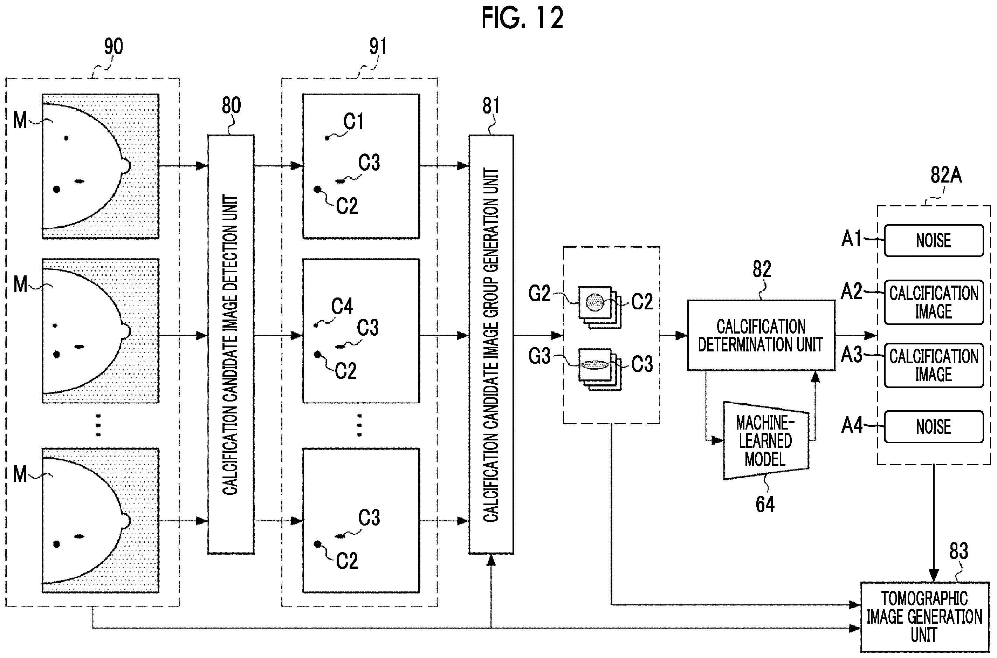

Fig. 12 schematically illustrates a flow of processing by the image processing apparatus 16 according to the first modification example. In the example illustrated in Fig. 12, the calcification candidate images C1 and C4 are detected from only one projection image 90, and the calcification candidate images C2 and C3 are detected from two or more projection images 90. Therefore, in the present example, the calcification candidate image group generation unit 81 generates only calcification candidate image groups G2 and G3 respectively including the calcification candidate images C2 and C3 among the calcification candidate images C1 to C4.

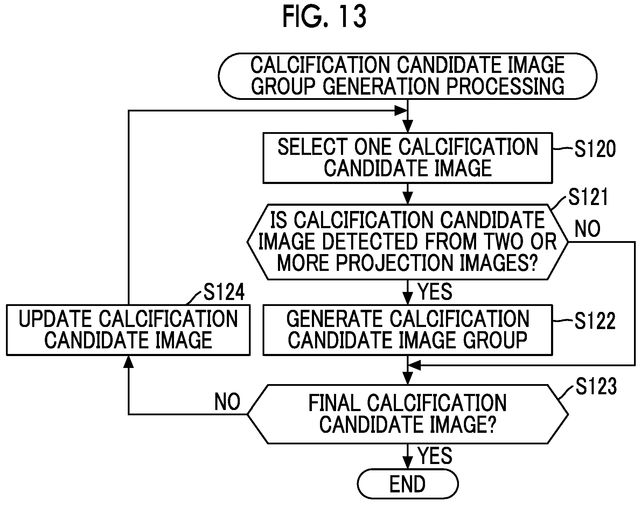

Fig. 13 illustrates a flow of calcification candidate image group generation processing according to the first modification example. First, in step S120, the calcification candidate image group generation unit 81 selects one calcification candidate image from among the plurality of calcification candidate images detected in the calcification candidate image detection processing.

In step S121, the calcification candidate image group generation unit 81 determines whether or not the calcification candidate image selected in step S120 is detected from two or more projection images 90. In a case where the calcification candidate image is detected from two or more projection images 90, a determination result is Yes, and the processing proceeds to step S122. In a case where the calcification candidate image is not detected from two or more projection images 90, a determination result is No, and the processing proceeds to step S123.

In step S122, the calcification candidate image group generation unit 81 generates a calcification candidate image group including the calcification candidate image detected from two or more projection images 90.

In step S123, the calcification candidate image group generation unit 81 determines whether or not the calcification candidate image selected in step S120 is a final calcification candidate image. That is, in step S123, the calcification candidate image group generation unit 81 determines whether or not the determination in step S121 is performed for all the calcification candidate images. In a case where the calcification candidate image is a final calcification candidate image, a determination result is Yes, and the processing is ended. In a case where the calcification candidate image is not a final calcification candidate image, a determination result is No, and the processing proceeds to step S124.

In step S124, the calcification candidate image group generation unit 81 changes the calcification candidate image to another calcification candidate image that is not selected in step S120. After step S124, the processing returns to step S120. Pieces of processing of step S120 to step S124 are repeatedly performed until the determination result in step S123 is Yes.

In the present modification example, the calcification candidate image detected from only one projection image 90 among the series of the plurality of projection images 90 is likely to be noise, and thus, the calcification candidate image group is not generated. In this way, the calcification candidate image detected from only one projection image 90 is excluded in advance from targets for generation of the calcification candidate image group, and thus the processing speed by the image processing apparatus 16 can be increased.

In step S121, the calcification candidate image group generation unit 81 determines whether or not the calcification candidate image selected in step S120 is detected from two or more projection images 90. On the other hand, the number of images as a determination criterion is not limited to two. That is, the calcification candidate image group generation unit 81 may determine whether or not the calcification candidate image is detected from a predetermined number or more of projection images 90.

### [Second Modification Example]

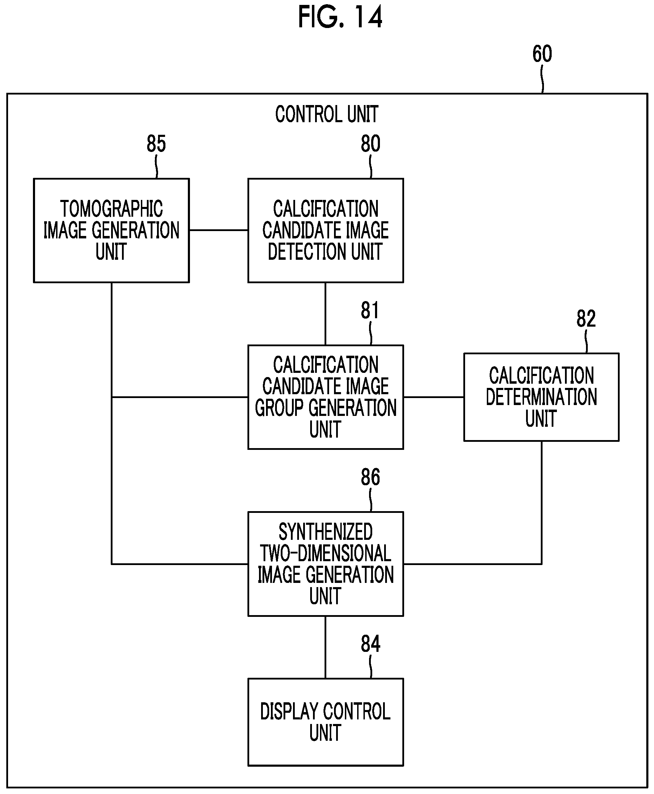

Fig. 14 illustrates a function realized by the control unit 60 of the image processing apparatus 16 according to a second modification example. In the present modification example, the control unit 60 functions as a tomographic image generation unit 85, a calcification candidate image detection unit 80, a calcification candidate image group generation unit 81, a calcification determination unit 82, a synthesized two-dimensional image generation unit 86, and a display control unit 84.

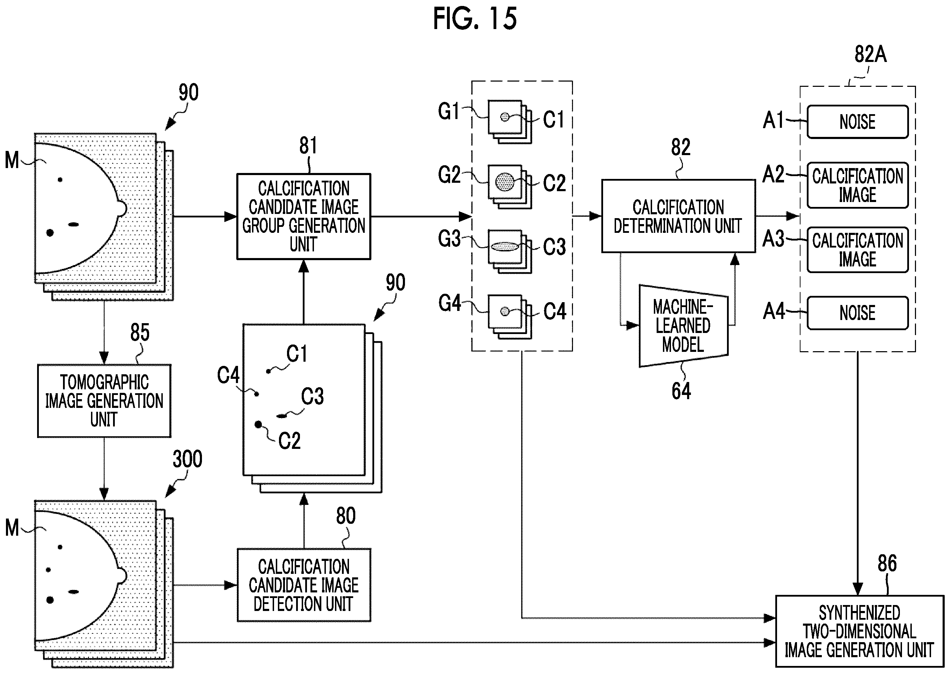

Fig. 15 schematically illustrates a flow of processing by the image processing apparatus 16 according to the second modification example. The tomographic image generation unit 85 acquires a series of a plurality of projection images 90 from the console 12 of the mammography apparatus 10 or the PACS 14. The tomographic image generation unit 85 generates a plurality of tomographic images 300 having different heights from the imaging surface 24A by a back projection method based on the series of the plurality of acquired projection images 90.

In the present modification example, the calcification candidate image detection unit 80 performs calcification candidate image detection processing of detecting, as a calcification candidate image, a region estimated to be a calcification image from the plurality of tomographic images 300. In the present modification example, the calcification candidate image detection unit 80 outputs a plurality of mask images 91 corresponding to each of the plurality of tomographic images 300. In the example illustrated in Fig. 15, four calcification candidate images C1 to C4 are detected by the calcification candidate image detection unit 80.

The calcification candidate image group generation unit 81 generates a calcification candidate image group including a plurality of calcification candidate images by cutting out a region corresponding to the same calcification candidate image, from each of the series of the plurality of projection images 90, based on the plurality of mask images 91 and the positional information of the radiation tube when each of the series of the plurality of projection images 90 is captured. In addition, in a case where a plurality of calcification candidate images are detected in the calcification candidate image detection processing, the calcification candidate image group generation unit 81 individually generates a calcification candidate image group for each of the calcification candidate images. In the example illustrated in Fig. 15, the calcification candidate image group generation unit 81 individually generates a calcification candidate image group for each of the four calcification candidate images C1 to C4.

The calcification determination unit 82 performs calcification determination processing of determining whether or not the calcification candidate image is a calcification image based on the calcification candidate image group generated by the calcification candidate image group generation processing, as in the above embodiment.

The synthesized two-dimensional image generation unit 86 performs weighting on the calcification candidate image based on the calcification determination result 82A obtained by the calcification determination processing, and then performs synthesized two-dimensional image generation processing of generating a synthesized two-dimensional image by synthesizing the plurality of tomographic images 300.

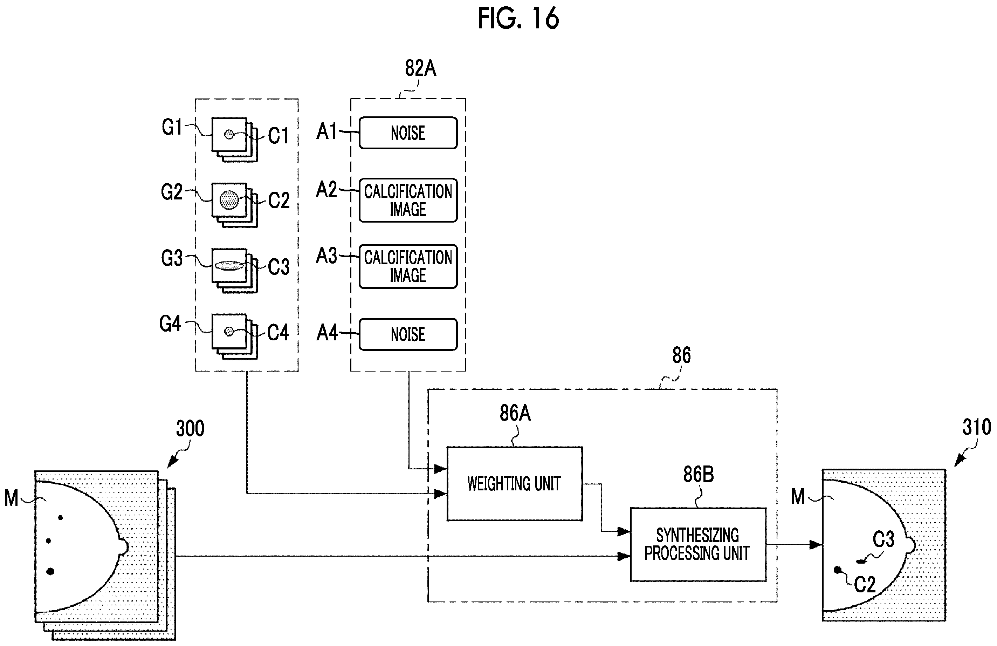

Fig. 16 conceptually illustrates an example of synthesized two-dimensional image generation processing by the synthesized two-dimensional image generation unit 86. As illustrated in Fig. 16, the synthesized two-dimensional image generation unit 86 includes a weighting unit 86A and a synthesizing processing unit 86B. The weighting unit 86A performs weighting on the calcification candidate image based on the calcification determination result 82A. Specifically, the weighting unit 86A increases a weight of a pixel included in the calcification candidate image determined to be a calcification image, and decreases a weight of a pixel included in the calcification candidate image determined to be noise.

The synthesizing processing unit 86B performs weighting on pixels of the plurality of tomographic images 300, which are generated by the tomographic image generation unit 85, by the weighting unit 86A, and then generates a synthesized two-dimensional image 310 by synthesizing the plurality of tomographic images 300. As a method of synthesizing the plurality of tomographic images 300, for example, an addition method, an average method, a maximum value projection method, a minimum value projection method, or the like can be used. The synthesizing processing unit 86B outputs the synthesized two-dimensional image 310 that is generated to the display control unit 84.

In the present modification example, the display control unit 84 performs display processing of displaying the synthesized two-dimensional image 310 generated by the synthesized two-dimensional image generation processing on the display unit 70. The display control unit 84 may perform highlight display in the synthesized two-dimensional image 310 by coloring or the like the calcification candidate image determined to be a calcification image by the calcification determination processing.

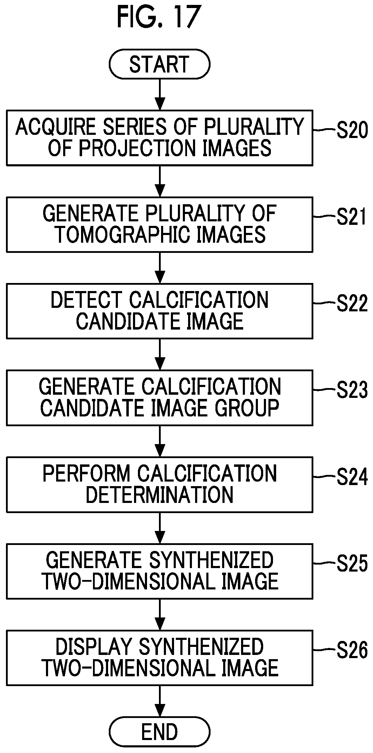

Next, a series of processing by the image processing apparatus 16 according to the second modification example will be described with reference to Fig. 17. First, in step S20, the tomographic image generation unit 85 acquires a series of a plurality of projection images 90 from the console 12 of the mammography apparatus 10 or the PACS 14.

In step S21, the tomographic image generation unit 85 generates a plurality of tomographic images 300 based on the plurality of projection images 90 acquired in step S20.

In step S22, the calcification candidate image detection unit 80 detects, as a calcification candidate image, a region estimated to be a calcification image from the plurality of tomographic images 300 generated in step S21.

In step S23, the calcification candidate image group generation unit 81 generates a calcification candidate image group by cutting out regions respectively corresponding to the region including the calcification candidate image detected in step S22, from each of the plurality of projection images 90.

In step S24, the calcification determination unit 82 determines whether or not the calcification candidate image is a calcification image based on the calcification candidate image group generated in step S23. Specifically, the calcification determination unit 82 inputs the calcification candidate image group to the machine-learned model 64, and acquires a calcification determination result 82A from the machine-learned model 64.

In step S25, the synthesized two-dimensional image generation unit 86 performs weighting on the calcification candidate image based on the calcification determination result 82A acquired in step S24, and then generates a synthesized two-dimensional image 310 by synthesizing the plurality of projection images 90.

In step S26, the display control unit 84 displays the synthesized two-dimensional image 310 generated in step S25 on the display unit 70.

Even in the present modification example, based on the calcification candidate image group generated by cutting out the regions respectively corresponding to the region including the calcification candidate image, from each of the plurality of projection images 90, it is determined whether or not the calcification candidate image is a calcification image, and thus, it is possible to accurately distinguish a calcification image and noise.

### [Other Modification Examples]

In the embodiment and each modification example, the calcification determination unit 82 performs the calcification determination processing using the machine-learned model 64. On the other hand, the calcification determination processing may be performed by a method such as image analysis, without using the machine-learned model 64. For example, the calcification determination unit 82 determines whether or not the calcification candidate image is a calcification image by analyzing a pattern of signals included in each of the calcification candidate images included in the calcification candidate image group. For example, as illustrated in Fig. 10, in a case where a common signal S is included in each of the calcification candidate images, the calcification determination unit 82 determines that the calcification candidate image corresponding to the signal S is a calcification image.

As described above, the calcification determination unit 82 may determine whether or not the calcification candidate image is a calcification image based on a pattern of signals included in each of the calcification candidate images included in the calcification candidate image group. The pattern of the signals that is to be analyzed by the calcification determination unit 82 may be a distribution of intensities of signals (for example, a variance value). In addition, the calcification determination unit 82 may determine whether or not the calcification candidate image is a calcification image based on a pattern of signals included in a small region of the calcification candidate image without being limited to the entire calcification candidate image.

The embodiment and the modification examples can be appropriately combined as long as there is no contradiction.

In addition, in the embodiment and the first modification example, as the hardware structure of a processing unit that executes various types of processing, such as the calcification candidate image detection unit 80, the calcification candidate image group generation unit 81, the calcification determination unit 82, the tomographic image generation unit 83, and the display control unit 84, the following various processors can be used. Further, in the second modification example, as the hardware structure of a processing unit that executes various types of processing, such as the tomographic image generation unit 85, the calcification candidate image detection unit 80, the calcification candidate image group generation unit 81, the calcification determination unit 82, the synthesized two-dimensional image generation unit 86, and the display control unit 84, the following various processors can be used.

The various processors include a graphics processing unit (GPU) in addition to a CPU. In addition, the various processors are not limited to a general-purpose processor such as a CPU that functions as various processing units by executing software (program), and include a dedicated electric circuit, which is a processor having a circuit configuration specifically designed to execute specific processing, such as a programmable logic device (PLD) or an application specific integrated circuit (ASIC) that is a processor of which the circuit configuration may be changed after manufacturing such as a field programmable gate array (FPGA).

One processing unit may be configured by one of the various processors, or may be configured by a combination of the same or different kinds of two or more processors (for example, a combination of a plurality of FPGAs or a combination of the CPU and the FPGA). In addition, a plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by a system on chip (SoC) or the like, a form in which a processor that realizes the function of the entire system including the plurality of processing units by one integrated circuit (IC) chip is used may be adopted. As described above, the various processing units are configured by using one or more various processors as a hardware structure.

Furthermore, as the hardware structure of the various processors, more specifically, an electrical circuit (circuitry) in which circuit elements such as semiconductor elements are combined can be used.

In addition, in the embodiment and the modification examples, a form in which the program 63 is stored in the storage unit 62 in advance has been described. On the other hand, the present disclosure is not limited thereto. The program 63 may be provided by being recorded in a non-transitory recording medium such as a compact disc read only memory (CD-ROM), a digital versatile disc read only memory (DVD-ROM), or a Universal Serial Bus (USB) memory. Further, the program 63 may be downloaded from an external apparatus via a network.

The described contents and the illustrated contents are the detailed description of the parts according to the technology of the present disclosure, and are merely examples of the technology of the present disclosure. For example, the descriptions related to the configuration, the function, the operation, and the effect are descriptions related to examples of a configuration, a function, an operation, and an effect of a part according to the technology of the present disclosure. Therefore, it goes without saying that, in the described contents and illustrated contents, unnecessary parts may be deleted, new components may be added, or replacements may be made without departing from the appended claims. Further, in order to avoid complications and facilitate understanding of the part according to the technology of the present disclosure, in the described contents and illustrated contents, descriptions of technical knowledge and the like that do not require particular explanations to enable implementation of the technology of the present disclosure are omitted.

## Claims

1. An image processing apparatus (16) comprising:
at least one processor (60),
wherein the processor is configured to execute:
calcification candidate image detection processing of detecting (S11, S22) a calcification candidate image (C1, C2, C3, C4) estimated to be a calcification image (A1, A2, A3, A4) from a series of a plurality of projection images (90) obtained by tomosynthesis imaging of a breast (M) or a plurality of tomographic images (100) obtained from the plurality of projection images;
calcification candidate image group generation processing of generating (S12, S23) a calcification candidate image group (G1, G2, G3, G4) by cutting out a region including the calcification candidate image detected by the calcification candidate image detection processing, from each of the plurality of projection images; and
calcification determination processing of determining (S13, S24) whether or not the calcification candidate image is a calcification image based on the calcification candidate image group generated by the calcification candidate image group generation processing,
wherein the processor is configured to:
in a case where a plurality of the calcification candidate images are detected in the calcification candidate image detection processing, individually generate the calcification candidate image group for each of the plurality of calcification candidate images in the calcification candidate image group generation processing.

2. The image processing apparatus (16) according to claim 1,
wherein the processor (60) is configured to:
detect (S11, S22) only the calcification candidate image (C1, C2, C3, C4) of which a signal value is equal to or smaller than a certain value in the calcification candidate image detection processing.

3. The image processing apparatus (16) according to claim 1 or 2,
wherein the processor (60) is configured to:
detect (S11, S22) the calcification candidate image (C1, C2, C3, C4) estimated to be a calcification image (A1, A2, A3, A4) from the plurality of projection images (90) in the calcification candidate image group generation processing; and
generate (S12, S23) the calcification candidate image group (G1, G2, G3, G4) in the calcification candidate image group generation processing, in a case where a corresponding calcification candidate image is detected from a certain number or more of the projection images among the series of the plurality of projection images in the calcification candidate image detection processing.

4. The image processing apparatus (16) according to claim 1 or 2,
wherein the processor (60) is configured to:
detect (S11, S22) the calcification candidate image (C1, C2, C3, C4) estimated to be a calcification image (A1, A2, A3, A4) from the plurality of projection images (90) in the calcification candidate image group generation processing; and
perform (S14) weighting on the calcification candidate image based on a determination result (82A) of the calcification determination processing, and generate the plurality of tomographic images by a back projection method.

5. The image processing apparatus (16) according to claim 1 or 2,
wherein the processor (60) is configured to:
detect (S22) the calcification candidate image (C1, C2, C3, C4) estimated to be a calcification image (A1, A2, A3, A4) from the plurality of tomographic images (100) in the calcification candidate image group generation processing; and
perform (S25) weighting on the calcification candidate image based on a determination result (82A) of the calcification determination processing, and generate a synthesized two-dimensional image (310) by synthesizing the plurality of tomographic images.

6. The image processing apparatus (16) according to any one of claims 1 to 5,
wherein the processor (60) is configured to:
execute (S13) the calcification determination processing by inputting the calcification candidate image group (G1, G2, G3, G4) into a machine-learned model (64) obtained by performing machine learning of a relationship between the calcification candidate image group and whether or not the calcification candidate image (C1, C2, C3, C4) is a calcification image (A1, A2, A3, A4).

7. The image processing apparatus (16) according to any one of claims 1 to 5,
wherein the processor (60) is configured to:
determine (S24) whether or not the calcification candidate image (C1, C2, C3, C4) is a calcification image (A1, A2, A3, A4) based on a pattern of signals included in each of the calcification candidate images included in the calcification candidate image group (G1, G2, G3, G4) in the calcification determination processing.

8. An image processing method comprising:
a calcification candidate image detection step of detecting (S11, S22) a calcification candidate image (C1, C2, C3, C4) estimated to be a calcification image (A1, A2, A3, A4) from a series of a plurality of projection images (90) obtained by tomosynthesis imaging of a breast (M) or a plurality of tomographic images (100) obtained from the plurality of projection images;
a calcification candidate image group generation step of generating (S12, S23) a calcification candidate image group (G1, G2, G3, G4) by cutting out a region including the calcification candidate image detected by the calcification candidate image detection step, from each of the plurality of projection images; and
a calcification determination step of determining (S13, S24) whether or not the calcification candidate image is a calcification image based on the calcification candidate image group generated by the calcification candidate image group generation step,
wherein in a case where a plurality of the calcification candidate images are detected in the calcification candidate image detection step, the calcification candidate image group for each of the plurality of calcification candidate images is individually generated in the calcification candidate image group generation step.

9. A computer-readable storage medium (62) storing a program (63) causing a computer (16) to execute:
calcification candidate image detection processing of detecting (S11, S22) a calcification candidate image (C1, C2, C3, C4) estimated to be a calcification image (A1, A2, A3, A4) from a series of a plurality of projection images (90) obtained by tomosynthesis imaging of a breast (M) or a plurality of tomographic images (100) obtained from the plurality of projection images;
calcification candidate image group generation processing of generating (S12, S23) a calcification candidate image group (G1, G2, G3, G4) by cutting out a region including the calcification candidate image detected by the calcification candidate image detection processing, from each of the plurality of projection images; and
calcification determination processing of determining (S13, S24) whether or not the calcification candidate image is a calcification image based on the calcification candidate image group generated by the calcification candidate image group generation processing,
wherein the program causes the computer to:
in a case where a plurality of the calcification candidate images are detected in the calcification candidate image detection processing, individually generate the calcification candidate image group for each of the plurality of calcification candidate images in the calcification candidate image group generation processing.

## Patentansprüche

1. Bildverarbeitungsvorrichtung (16), umfassend:
mindestens einen Prozessor (60),
wobei der Prozessor so konfiguriert ist, dass er ausführt:
eine Detektionsverarbeitung für Verkalkungskandidatenbild zum Detektieren (S11, S22) eines Verkalkungskandidatenbildes (C1, C2, C3, C4), das als ein Verkalkungsbild (A1, A2, A3, A4) geschätzt wird, aus einer Reihe von mehreren Projektionsbildern (90), die durch Tomosynthesebildgebung einer Brust (M) erhalten werden, oder mehreren Tomographiebildern (100), die aus den mehreren Projektionsbildern erhalten werden;
eine Erzeugungsverarbeitung für Verkalkungskandidatenbild-Gruppe zum Erzeugen (S12, S23) einer Verkalkungskandidatenbild-Gruppe (G1, G2, G3, G4) durch Ausschneiden eines Bereichs, der das von der Detektionsverarbeitung für Verkalkungskandidatenbild detektierte Verkalkungskandidatenbild enthält, aus jedem der mehreren Projektionsbilder; und
eine Bestimmungsverarbeitung für Verkalkung zum Bestimmen (S13, S24), ob das Verkalkungskandidatenbild ein Verkalkungsbild ist oder nicht, auf der Grundlage der von der Erzeugungsverarbeitung für Verkalkungskandidatenbild-Gruppe erzeugten Verkalkungskandidatenbild-Gruppe,
wobei der Prozessor so konfiguriert ist, dass er:
in einem Fall, in dem mehrere der Verkalkungskandidatenbilder bei der Detektionsverarbeitung für Verkalkungskandidatenbild detektiert werden, die Verkalkungskandidatenbild-Gruppe für jedes der mehreren Verkalkungskandidatenbilder bei der Erzeugungsverarbeitung für Verkalkungskandidatenbild-Gruppe individuell erzeugt.

2. Bildverarbeitungsvorrichtung (16) nach Anspruch 1,
wobei der Prozessor (60) so konfiguriert ist, dass er:
bei der Detektionsverarbeitung für Verkalkungskandidatenbild nur das Verkalkungskandidatenbild (C1, C2, C3, C4), dessen Signalwert gleich oder kleiner als ein bestimmter Wert ist, detektiert (S11, S22).

3. Bildverarbeitungsvorrichtung (16) nach Anspruch 1 oder 2,
wobei der Prozessor (60) so konfiguriert ist, dass er:
bei der Erzeugungsverarbeitung für Verkalkungskandidatenbild-Gruppe das Verkalkungskandidatenbild (C1, C2, C3, C4), das als ein Verkalkungsbild (A1, A2, A3, A4) geschätzt wird, aus den mehreren Projektionsbildern (90) detektiert (S11, S22); und
die Verkalkungskandidatenbild-Gruppe (G1, G2, G3, G4) bei der Erzeugungsverarbeitung für Verkalkungskandidatenbild-Gruppe in einem Fall, in dem ein entsprechendes Verkalkungskandidatenbild aus einer bestimmten Anzahl oder mehr der Projektionsbilder unter den Reihen der mehreren Projektionsbilder bei der Detektionsverarbeitung für Verkalkungskandidatenbild detektiert wird, erzeugt (S12, S23).

4. Bildverarbeitungsvorrichtung (16) nach Anspruch 1 oder 2,
wobei der Prozessor (60) so konfiguriert ist, dass er:
bei der Erzeugungsverarbeitung für Verkalkungskandidatenbild-Gruppe das Verkalkungskandidatenbild (C1, C2, C3, C4), das als ein Verkalkungsbild (A1, A2, A3, A4) geschätzt wird, aus den mehreren Projektionsbildern (90) detektiert (S11, S22); und
Gewichtung an dem Verkalkungskandidatenbild auf der Grundlage eines Bestimmungsergebnisses (82A) der Bestimmungsverarbeitung für Verkalkung durchführt (S14) und die mehreren Tomographiebilder durch ein Rückprojektionsverfahren erzeugt.

5. Bildverarbeitungsvorrichtung (16) nach Anspruch 1 oder 2,
wobei der Prozessor (60) so konfiguriert ist, dass er:
bei der Erzeugungsverarbeitung für Verkalkungskandidatenbild-Gruppe das Verkalkungskandidatenbild (C1, C2, C3, C4), das als ein Verkalkungsbild (A1, A2, A3, A4) geschätzt wird, aus den mehreren Tomographiebildern (100) detektiert (S22); und
Gewichtung an dem Verkalkungskandidatenbild auf der Grundlage eines Bestimmungsergebnisses (82A) der Bestimmungsverarbeitung für Verkalkung durchführt (S25) und ein synthetisiertes zweidimensionales Bild (310) durch Synthetisieren der mehreren Tomographiebilder erzeugt.

6. Bildverarbeitungsvorrichtung (16) nach einem der Ansprüche 1 bis 5,
wobei der Prozessor (60) so konfiguriert ist, dass er:
die Bestimmungsverarbeitung für Verkalkung ausführt (S13), indem die Verkalkungskandidatenbild-Gruppe (G1, G2, G3, G4) in ein maschinell gelerntes Modell (64) eingegeben wird, das durch Durchführen von maschinellem Lernen einer Beziehung zwischen der Verkalkungskandidatenbild-Gruppe und dem, ob das Verkalkungskandidatenbild (C1, C2, C3, C4) ein Verkalkungsbild (A1, A2, A3, A4) ist oder nicht, erhalten wird.

7. Bildverarbeitungsvorrichtung (16) nach einem der Ansprüche 1 bis 5,
wobei der Prozessor (60) so konfiguriert ist, dass er:
bei der Bestimmungsverarbeitung für Verkalkung auf der Grundlage eines Musters von Signalen, die in jedem der Verkalkungskandidatenbilder, die in der Verkalkungskandidatenbild-Gruppe (G1, G2, G3, G4) enthalten sind, enthalten sind, bestimmt (S24), ob das Verkalkungskandidatenbild (C1, C2, C3, C4) ein Verkalkungsbild (A1, A2, A3, A4) ist oder nicht.

8. Bildverarbeitungsverfahren, umfassend:
einen Detektionsschritt für Verkalkungskandidatenbild des Detektierens (S11, S22) eines Verkalkungskandidatenbildes (C1, C2, C3, C4), das als ein Verkalkungsbild (A1, A2, A3, A4) geschätzt wird, aus einer Reihe von mehreren Projektionsbildern (90), die durch Tomosynthesebildgebung einer Brust (M) erhalten werden, oder mehreren Tomographiebildern (100), die aus den mehreren Projektionsbildern erhalten werden;
einen Erzeugungsschritt für Verkalkungskandidatenbild-Gruppe des Erzeugens (S12, S23) einer Verkalkungskandidatenbild-Gruppe (G1, G2, G3, G4) durch Ausschneiden eines Bereichs, der das durch den Verkalkungskandidatenbild-Detektionsschritt detektierte Verkalkungskandidatenbild enthält, aus jedem der mehreren Projektionsbilder; und
einen Bestimmungsschritt für Verkalkung des Bestimmens (S13, S24), ob das Verkalkungskandidatenbild ein Verkalkungsbild ist oder nicht, auf der Grundlage der Verkalkungskandidatenbild-Gruppe, die durch den Erzeugungsschritt für Verkalkungskandidatenbild-Gruppe erzeugt wird,
wobei in einem Fall, in dem mehrere der Verkalkungskandidatenbilder bei dem Detektionsschritt für Verkalkungskandidatenbild detektiert werden, die Verkalkungskandidatenbild-Gruppe für jedes der mehreren Verkalkungskandidatenbilder bei dem Erzeugungsschritt für Verkalkungskandidatenbild-Gruppe individuell erzeugt wird.

9. Computerlesbares Speichermedium (62), das ein Programm (63) speichert, das einen Computer (16) veranlasst, auszuführen:
eine Detektionsverarbeitung für Verkalkungskandidatenbild zum Detektieren (S11, S22) eines Verkalkungskandidatenbildes (C1, C2, C3, C4), das als ein Verkalkungsbild (A1, A2, A3, A4) geschätzt wird, aus einer Reihe von mehreren Projektionsbildern (90), die durch Tomosynthesebildgebung einer Brust (M) erhalten werden, oder mehreren Tomographiebildern (100), die aus den mehreren Projektionsbildern erhalten werden;
eine Erzeugungsverarbeitung für Verkalkungskandidatenbild-Gruppe zum Erzeugen (S12, S23) einer Verkalkungskandidatenbild-Gruppe (G1, G2, G3, G4) durch Ausschneiden eines Bereichs, der das von der Detektionsverarbeitung für Verkalkungskandidatenbild detektierte Verkalkungskandidatenbild enthält, aus jedem der mehreren Projektionsbilder; und
eine Bestimmungsverarbeitung für Verkalkung zum Bestimmen (S13, S24), ob das Verkalkungskandidatenbild ein Verkalkungsbild ist oder nicht, auf der Grundlage der von der Erzeugungsverarbeitung für Verkalkungskandidatenbild-Gruppe erzeugten Verkalkungskandidatenbild-Gruppe,
wobei das Programm den Computer veranlasst:
in einem Fall, in dem mehrere der Verkalkungskandidatenbilder bei der Detektionsverarbeitung für Verkalkungskandidatenbild detektiert werden, die Verkalkungskandidatenbild-Gruppe für jedes der mehreren Verkalkungskandidatenbilder bei der Erzeugungsverarbeitung für Verkalkungskandidatenbild-Gruppe individuell zu erzeugen.

## Revendications

1. Appareil de traitement d'images (16) comprenant :
au moins un processeur (60),
dans lequel le processeur est configuré pour exécuter :
un traitement de détection d'images candidates à une calcification consistant à détecter (S11, S22) une image candidate à une calcification (C1, C2, C3, C4) estimée être une image de calcification (A1, A2, A3, A4) à partir d'une série d'une pluralité d'images de projection (90) obtenues par imagerie par tomosynthèse d'un sein (M) ou d'une pluralité d'images tomographiques (100) obtenues à partir de la pluralité d'images de projection ;
un traitement de génération de groupe d'images candidates à une calcification consistant à générer (S12, S23) un groupe d'images candidates à une calcification (G1, G2, G3, G4) en découpant une région incluant l'image candidate à une calcification détectée par le traitement de détection d'images candidates à une calcification, à partir de chacune de la pluralité d'images de projection ; et
un traitement de détermination de calcification consistant à déterminer (S13, S24) si oui ou non l'image candidate à une calcification est une image de calcification sur la base du groupe d'images candidates à une calcification généré par le traitement de génération de groupe d'images candidates à une calcification,
dans lequel le processeur est configuré pour :
dans un cas où une pluralité d'images candidates à une calcification sont détectées dans le traitement de détection d'images candidates à une calcification, générer individuellement le groupe d'images candidates à une calcification pour chacune de la pluralité d'images candidates à une calcification dans le traitement de génération de groupe d'images candidates à une calcification.

2. Appareil de traitement d'images (16) selon la revendication 1,
dans lequel le processeur (60) est configuré pour :
détecter (S11, S22) uniquement l'image candidate à une calcification (C1, C2, C3, C4) dont une valeur de signal est égale ou inférieure à une certaine valeur dans le traitement de détection d'images candidates à une calcification.

3. Appareil de traitement d'images (16) selon la revendication 1 ou la revendication 2,
dans lequel le processeur (60) est configuré pour :
détecter (S11, S22) l'image candidate à une calcification (C1, C2, C3, C4) estimée être une image de calcification (A1, A2, A3, A4) à partir de la pluralité d'images de projection (90) dans le traitement de génération de groupe d'images candidates à une calcification ; et
générer (S12, S23) le groupe d'images candidates à une calcification (G1, G2, G3, G4) dans le traitement de génération de groupe d'images candidates à une calcification, dans un cas où une image candidate à une calcification correspondante est détectée à partir d'un certain nombre ou plus des images de projection parmi la série de la pluralité d'images de projection dans le traitement de détection d'images candidates à une calcification.

4. Appareil de traitement d'images (16) selon la revendication 1 ou la revendication 2,
dans lequel le processeur (60) est configuré pour :
détecter (S11, S22) l'image candidate à une calcification (C1, C2, C3, C4) estimée être une image de calcification (A1, A2, A3, A4) à partir de la pluralité d'images de projection (90) dans le traitement de génération de groupe d'images candidates à une calcification ; et
effectuer (S14) une pondération sur l'image candidate à une calcification sur la base d'un résultat de détermination (82A) du traitement de détermination de calcification, et générer la pluralité d'images tomographiques par une méthode de rétroprojection.

5. Appareil de traitement d'images (16) selon la revendication 1 ou la revendication 2,
dans lequel le processeur (60) est configuré pour :
détecter (S22) l'image candidate à une calcification (C1, C2, C3, C4) estimée être une image de calcification (A1, A2, A3, A4) à partir de la pluralité d'images tomographiques (100) dans le traitement de génération de groupe d'images candidates à une calcification ; et
effectuer (S25) une pondération sur l'image candidate à une calcification sur la base d'un résultat de détermination (82A) du traitement de détermination de calcification, et générer une image bidimensionnelle synthétisée (310) en synthétisant la pluralité d'images tomographiques.

6. Appareil de traitement d'images (16) selon l'une quelconque des revendications 1 à 5,
dans lequel le processeur (60) est configuré pour :
exécuter (S13) le traitement de détermination de calcification en entrant le groupe d'images candidates à une calcification (G1, G2, G3, G4) dans un modèle appris par apprentissage automatique (64) obtenu en effectuant un apprentissage automatique d'une relation entre le groupe d'images candidates à une calcification et si oui ou non l'image candidate à une calcification (C1, C2, C3, C4) est une image de calcification (A1, A2, A3, A4).

7. Appareil de traitement d'images (16) selon l'une quelconque des revendications 1 à 5,
dans lequel le processeur (60) est configuré pour :
déterminer (S24) si oui ou non l'image candidate à une calcification (C1, C2, C3, C4) est une image de calcification (A1, A2, A3, A4) sur la base d'un motif de signaux inclus dans chacune des images candidates à une calcification incluses dans le groupe d'images candidates à une calcification (G1, G2, G3, G4) dans le traitement de détermination de calcification.

8. Procédé de traitement d'images comprenant :
une étape de détection d'images candidates à une calcification consistant à détecter (S11, S22) une image candidate à une calcification (C1, C2, C3, C4) estimée être une image de calcification (A1, A2, A3, A4) à partir d'une série d'une pluralité d'images de projection (90) obtenues par imagerie par tomosynthèse d'un sein (M) ou d'une pluralité d'images tomographiques (100) obtenues à partir de la pluralité d'images de projection ;
une étape de génération de groupe d'images candidates à une calcification consistant à générer (S12, S23) un groupe d'images candidates à une calcification (G1, G2, G3, G4) en découpant une région incluant l'image candidate à une calcification détectée par l'étape de détection d'images candidates à une calcification, à partir de chacune de la pluralité d'images de projection ; et
une étape de détermination de calcification consistant à déterminer (S13, S24) si oui ou non l'image candidate à une calcification est une image de calcification sur la base du groupe d'images candidates à une calcification généré par l'étape de génération de groupe d'images candidates à une calcification,
dans lequel dans un cas où une pluralité d'images candidates à une calcification sont détectées dans l'étape de détection d'images candidates à une calcification, le groupe d'images candidates à une calcification pour chacune de la pluralité d'images candidates à une calcification est généré individuellement dans l'étape de génération de groupe d'images candidates à une calcification.

9. Support de stockage lisible par ordinateur (62) stockant un programme (63) amenant un ordinateur (16) à exécuter :
un traitement de détection d'images candidates à une calcification consistant à détecter (S11, S22) une image candidate à une calcification (C1, C2, C3, C4) estimée être une image de calcification (A1, A2, A3, A4) à partir d'une série d'une pluralité d'images de projection (90) obtenues par imagerie par tomosynthèse d'un sein (M) ou d'une pluralité d'images tomographiques (100) obtenues à partir de la pluralité d'images de projection ;
un traitement de génération de groupe d'images candidates à une calcification consistant à générer (S12, S23) un groupe d'images candidates à une calcification (G1, G2, G3, G4) en découpant une région incluant l'image candidate à une calcification détectée par le traitement de détection d'images candidates à une calcification, à partir de chacune de la pluralité d'images de projection ; et
un traitement de détermination de calcification consistant à déterminer (S13, S24) si oui ou non l'image candidate à une calcification est une image de calcification sur la base du groupe d'images candidates à une calcification généré par le traitement de génération de groupe d'images candidates à une calcification,
dans lequel le programme amène l'ordinateur à :
dans un cas où une pluralité d'images candidates à une calcification sont détectées dans le traitement de détection d'images candidates à une calcification, générer individuellement le groupe d'images candidates à une calcification pour chacune de la pluralité d'images candidates à une calcification dans le traitement de génération de groupe d'images candidates à une calcification.
